# EUROPEAN PATENT SPECIFICATION

(11) **EP 3 970 751 B1**
(45) Date of publication and mention of the grant of the patent: **23.08.2023**
(21) Application number: 20197479.7
(22) Date of filing: 22.09.2020
(51) Int. Cl.: A61K 47/02, A61K 47/10, A61K 47/36

(54) **OPHTHALMIC COMPOSITION FOR THE TREATMENT OF CORNEAL EDEMA**
OPHTHALMISCHE ZUSAMMENSETZUNG ZUR BEHANDLUNG VON HORNHAUTÖDEM
COMPOSITION OPHTALMIQUE POUR LE TRAITEMENT DE L' OEDÈME DE LA CORNÉE

(43) Date of publication of application: 23.03.2022
(73) Proprietor: OmniVision GmbH, 82178 Puchheim (DE)
(72) Inventor: KOHL, Tobias, 82178 Puchheim (DE); HOFFMANN, Burkhardt, 82178 Puchheim (DE); HOFFMANN, Patrick, 82178 Puchheim (DE)
(74) Representative: Blodig, Wolfgang

(56) References cited:
- US-A1- 2017 128 484
- G. HO WANG YIN ET AL: "Acuité visuelle, pachymétrie et densité cornéenne après traitement par solution hyperosmolaire au chlorure de sodium 5 % dans l'oedème cornéen postopératoire", JOURNAL FRANCAIS D'OPHTALMOLOGIE., vol. 38, no. 10, 5 November 2015 (2015-11-05), pages 967-973, XP55611051, FR ISSN: 0181-5512, DOI: 10.1016/j.jfo.2015.07.002
- Ursapharm: "Hylo-parin", , 1 November 2013 (2013-11-01), XP002802354, Retrieved from the Internet: URL:https://www.deonlinedrogist.nl/meer_in fo/PB-H-PARIN-BENELUX_LR1.pdf [retrieved on 2021-03-11]
- PASQUALE ARAGONA ET AL: "Physicochemical Properties of Hyaluronic Acid-Based Lubricant Eye Drops", TRANSLATIONAL VISION SCIENCE & TECHNOLOGY, vol. 8, no. 6, 1 November 2019 (2019-11-01), page 2, XP055755186, ISSN: 2164-2591, DOI: 10.1167/tvst.8.6.2

## Description

### FIELD OF THE INVENTION

The invention relates to an ophthalmic composition, notably an aqueous hyperosmolar ophthalmic composition, suitable for the treatment of, inter alia, corneal edema. The invention also provides an ophthalmic composition, notably an aqueous hyperosmolar ophthalmic composition, for use in a method of treating or reducing corneal swelling or fluid accumulation, including stromal corneal edema or epithelial corneal edema, especially after surgical intervention, such as cataract surgery or corneal transplantation; or for restoring osmotic balance of the cornea, especially after surgical intervention, such as cataract surgery or corneal transplantation.

### BACKGROUND OF THE INVENTION

Corneal edema is the swelling of the cornea following ocular surgery, trauma, infection, inflammation as well as a secondary result of various ocular diseases. Corneal edema can also occur following over-wear of certain types of contact lenses. Corneal edema lead to a thickening of the cornea. The cornea is part of the eye's focusing system that transmits and focuses light into the eye. When the cornea thickens, it may impair transmission of light, which may decrease vision. The thickening of the cornea can also lead to a loss of corneal transparency and a lowering of the visual acuity.

Corneal edema may be treated by topical application of hypertonic eye drops, generally containing hyperosmolar concentrations of sodium chloride. US 2017/0128484 A1 (Horus Pharma) describes an aqueous hyperosmolar solution containing hydroxypropylmethylcellulose (HPMC) and hyaluronic acid. The exemplified solution contains 5 % by weight sodium chloride, 0.25 % by weight HPMC, 0.15 % by weight sodium hyaluronate and citrate buffer. The solution is said to have a pH between 6.2 and 6.8 and an osmolality of about 1750 mOsm/kg. A commercial product of this type is ODM5^{™} of Horus Pharma.

The osmolarity of tears is generally within the range of about 280 to 330 mOsm/l. Thus, eye drops having an osmolarity or osmolality of a solution containing about 5 % by weight sodium chloride is non-physiological to the eye, notably to the cornea and leads to a strong burning sensation upon application to the eye. Moreover, upon application to the eye, cells of the corneal layers, notably cells of the outer epithelial layer, suffer an osmotic shock, which can potentially be damaging to corneal tissue.

Departing from the prior art, it is an object of the invention to provide an ophthalmic composition for the treatment of corneal edema that has, on the one hand, a high osmolarity or osmolality for the treatment of corneal edema and, on the other hand, no or little damaging effect on corneal tissue. It is another object of the invention to provide an ophthalmic composition for the treatment of corneal edema, that leads to low or reduced burning sensation upon application to the eye.

### SUMMARY OF THE INVENTION

The scope of the invention is defined by the claims. Any references in the description to method of treatment refer to the products of the present invention for use in a method of treatment. In order to accomplish these objects, the invention provides:
(1) A hyperosmolar ophthalmic composition comprising:
   (i) at least one osmolarity agent,
   (ii) heparin or a salt thereof in an amount of from 1000 to 1600 I.U. / mL of the ophthalmic composition, and
   (iii) at least one viscosity regulating agent,
      wherein
      (A) the total osmolality of the ophthalmic composition is at least 1500 mOsm/L preferably at least 1800 mOsm/L, or
      (B) the ophthalmic composition contains, as osmolarity agent (i), at least 4.4% by weight sodium chloride.
(2) The ophthalmic composition according to item 1, wherein the (i) at least one osmolarity agent of (A) is selected from the group consisting of sodium chloride, potassium chloride, trehalose, and sugar alcohols.
(3) The ophthalmic composition according to item 2, wherein the sugar alcohol is selected from the group consisting of glycerol, mannitol, and sorbitol.
(4) The ophthalmic composition according to any one of items 1 to 3, wherein the (i) at least one osmolarity agent is or comprises sodium chloride and one or more sugar alcohol.
(5) The ophthalmic composition according to any one of items 2 to 4, wherein the sugar alcohol is glycerol, preferably wherein the ophthalmic composition comprises sodium chloride and glycerol as osmolarity agents (i).
(6) The ophthalmic composition according to any one of items 1 to 5, comprising, as osmolarity agents (i), sodium chloride in a concentration of from 4.4 to 6.5 % by weight, preferably from 4.6 to 6.0 % by weight, more preferably from 4.8 to 5.5 % by weight, and even more preferably about 5 % by weight.
(7) The ophthalmic composition according to any one of items 1 to 6, comprising, as osmolarity agent (i), glycerol in an amount of from 1.5 to 3.5 % by weight, preferably from 2.0 to 3.0 % by weight, and even more preferably from 2.3 to 2.7 % by weight.
(8) The ophthalmic composition according to any one of items 1 to 7, containing, as component (ii), heparin or a salt thereof in an amount of from 1100 to 1500 I.U. / mL, preferably of from 1200 to 1400 I.U / mL, more preferably from 1250 to 1350 I.U. / mL of the ophthalmic composition.
(9) The ophthalmic composition according to any one of items 1 to 8, wherein the (iii) one or more viscosity regulating agent is selected from the group consisting of a hydroxyalkylated cellulose, chondroitin sulfate, polyacrylic acid, polyvinyl alcohol, polyethylene glycol, polysaccharides, polyvinylpyrrolidone, hyaluronic acid, cross-linked hyaluronic acid, and salts thereof, preferably wherein the (iii) one or more viscosity regulating agent is or comprises hyaluronic acid and/or a hyaluronate.
(10) The ophthalmic composition according to any one of items 1 to 9, comprising said (iii) one or more viscosity regulating agent, preferably said hyaluronic acid and/or hyaluronate, in a concentration of from 0.1 to 0.5 % by weight, preferably from 0.15 to 0.45% by weight, more preferably from 0.2 to 0.4 % by weight, and most preferably from 0.25 to 0.35% by weight.
(11) The ophthalmic composition according to any one of items 1 to 10, further comprising (iv) a buffer, preferably wherein the (iv) buffer is selected from the group consisting of a tris (trometamol) buffer and citrate buffer, preferably the buffer is tris buffer.
(12) The ophthalmic composition according to any one of items 1 to 11, having a pH of from 6.8 to 7.6; and/or a dynamic viscosity of from 20 to 70 cP (mPa s) at 20°C using a rational viscosimeter.
(13) An ophthalmic composition according any one of the preceding items for use in ophthalmology, preferably for use in a method of treating or reducing corneal swelling or fluid accumulation of any origin, including after cataract surgery or corneal transplantation, stromal corneal edema or epithelial corneal edema, or for restoring osmotic balance of the cornea, especially after surgical intervention such as cataract surgery or corneal transplantation.

The inventors have surprisingly found an ophthalmic composition that has a high osmolarity (and osmolality) e.g. for treatment of corneal edema, and provides a high and/or improved viability to corneal cells or tissue. The inventors have also found an ophthalmic composition that, despite of high osmolarity, is good in terms of the burning sensation upon topical application of the composition to the eye surface.

### DETAILED DESCRIPTION OF THE INVENTION

The hyperosmolar ophthalmic composition of the present invention is generally an aqueous hyperosmolar ophthalmic composition. The present invention provides, in a first embodiment, a hyperosmolar ophthalmic composition comprising components (i) to (iii) to be described in the following. The ophthalmic composition of the present invention generally has an osmolarity of at least 1500 mOsm/mL, preferably at least 1800 mOsm/mL, more preferably at least 2100 mOsm/L.

The present invention also provides, in a second embodiment, a hyperosmolar ophthalmic composition comprising components (i) to (iii) to be described in the following and, as osmolarity agent, at least 4.4 % by weight sodium chloride. The ophthalmic composition according the second embodiment comprises preferably (i,a) at least 4.4 % by weight sodium chloride and (i,b) glycerol in an amount from 1.5 to 3.5% by weight as osmolarity agents (i).

The term "ophthalmic composition" refers to a composition suitable for use in ophthalmology, notably suitable for topical application to the surface of the eyes. The ophthalmic composition of the invention is generally aqueous because it contains water. Examples of the ophthalmic composition are solutions, emulsions, and dispersions. Generally, the hyperosmolar ophthalmic composition of the invention is a solution of the components mentioned in water. Examples of the ophthalmic compositions are eye drops, ophthalmic gels, ophthalmic creams, and ophthalmic ointments. Preferred are eye drops, ophthalmic gels, and ophthalmic creams. Eye drops are most preferred.

Herein, the term "hyperosmolar" relates to aqueous compositions having an osmolarity or osmolality above that generally found in tears, i.e. above 300 mOsm/L or 300 mOsm/kg. However, the osmolarity of the hyperosmolar ophthalmic compositions of the invention is, as described in further detail below, generally much higher than 300 mOsm/L.

Osmolarity is the concentration of solute(s) in a solution and is defined as the number of osmoles (Osm) of solute per liter of solution in unit Osm/L or mOsm/L. Herein, the unit mOsm/L is mostly used. Osmolality is the concentration of solutes in a solution and is defined as the number of osmoles (Osm) of solute per kilogram of solvent in unit Osm/kg or mOsm/kg.

The osmole (Osm) is a unit of measurement which defines the number of moles of solute(s) that contribute(s) to the osmotic pressure of a solution. The osmolality (and osmolarity) of small molecules can generally be calculated from their concentration in solution. In the case of simple salts, such as sodium chloride, which dissociate completely in water, both the (sodium) cation and the (chloride) anion contribute to the osmolality and osmolarity which can be calculated approximately from the sum of the concentrations of cations and anions. In the case of complex salts such as sodium phosphate, which partially dissociate in water, the osmolality and osmolarity depend on concentration and pH. Therefore, herein, osmolality is determined experimentally, and numerical values of osmolality are measured values according to Ph. Eur. 2.2.35.

Osmolality and osmolarity of a given aqueous composition are linked by the density of the composition and the concentration of the solute: osmolarity = osmolality × (density of solution (kg/L) - mass concentration of solutes (kg/L)). Herein, the osmolarity of a solution is determined at a temperature of 25°C, unless otherwise specified.

The term "osmolarity agent" (may also be referred to as "hyperosmolarity agent" or "tonicity agent") generally refers to a substance that dissolves in the water of the composition of the invention and thus contributes to the osmolarity of the composition. Herein, the at least one osmolarity agent contributes the major part of the osmolarity to the composition, e.g. at least 90%, preferably at least 95%, and more preferably at least 97% of the osmolarity. The term "osmolarity agent" comprises both penetrating and non-penetrating solutes, i.e. solutes that can and that cannot penetrate a membrane of an osmotic cell. Examples of osmolarity agents are inorganic soluble salts such as sodium and potassium salts (e.g. sodium chloride, potassium chloride), mono- and disaccharides (e.g. trehalose), and sugar alcohols. Examples of sugar alcohols are glycerol, mannitol, xylitol, and sorbitol. One osmolarity agent may be used alone in the ophthalmic composition of the invention, or two or more may be used in combination. For example, inorganic soluble salts and sugar alcohols may be used in combination as the at least one osmolarity agent. Preferably, the at least one osmolarity agent is or comprises sodium chloride and one or more sugar alcohol. More preferably, the at least one osmolarity agent is or comprises sodium chloride and glycerol.

The at least one osmolarity agent should be present in the ophthalmic composition of the invention so as to achieve the desired osmolarity. The osmolarity may be at least 1500 mOsm/L, preferably at least 1800 mOsm/L, more preferably at least 2000 mOsm/L, and most preferably at least 2150 mOsm/L. The upper limit of the osmolarity is not specifically limited. However, it is generally not more than 2500, preferably not more than 2400 mOsm/L to avoid excessive osmotic shock and/or burning sensation upon application to an eye.

If one of the at least one osmolarity agent is sodium chloride, the sodium chloride may be present in a concentration of from 4.4 to 6.5% by weight, preferably from 4.6 to 6.0 % by weight, more preferably from 4.8 to 5.5 % by weight, and even more preferably about 5 % by weight. A content of the sodium chloride exceeding 6.5 % by weight is not preferred, since such content may cause excessive dehydration of ocular tissue such as the cornea, and a content of the sodium chloride below 4.4 % by weight is not preferred since such content may result in insufficient osmolarity for the desired dehydration effect. Within the ranges mentioned, eye irritation is suppressed to acceptable limits and good therapeutic effect can be achieved.

As mentioned above, the at least one osmolarity agent may be a combination of sodium chloride and a sugar alcohol, notably glycerol. The sugar alcohol may be present, in addition to the concentration of the sodium chloride, in a concentration of from 1.5 to 3.5 % by weight, preferably from 2.0 to 3.0 % by weight, and even more preferably from 2.3 to 2.7 % by weight of the ophthalmic composition. A content of the glycerol exceeding 3.5 % by weight is not preferred, since such content can cause excessive dehydration of ocular tissue, and a content of the glycerol below 1.5 % by weight is not preferred if such content results in insufficient osmolarity for the dehydration effect. Within the ranges mentioned, sufficiently high osmolarity may be achieved while an excessive burning effect or eye irritation upon application of the ophthalmic composition to the eye can be avoided.

Heparin is a sulfated glycosaminoglycan and thus a mucopolysaccharide. It is generally obtained from mucosal tissues of slaughtered animals, and is commercially available from various sources. The heparin for use in the invention may be unfractionated heparin, a low molecular weight heparin, an ultra-low molecular weight heparin, or a combination thereof. Examples of a low molecular weight heparin includes Bemiparin, Nadroparin, Reviparin, Enoxaparin, Parnaparin, Certoparin, Dalteparin, and Tinzparin. A mucopolysaccharide with heparin activity other than heparin may be sulfation modified heparin, a glycol-split heparin, glycol-split N-acetylated heparin, other heparin derivative, heparan sulfate, or a combination thereof. Preferably, the heparin for use in the present invention is selected from unfractionated heparin and low molecular weight heparin. Commercial sources for heparin sodium are, for example, Welding GmbH & Co KG, Midas Pharma GmbH, and Helm Portugal LDA.

As is common in the art, the amount or concentration of the heparin or other mucopolysaccharide with heparin activity is defined in terms of the International units (I.U.) defined by the WHO based on its biological activity: one unit of heparin is the quantity of heparin required to keep one ml of cat's blood fluid for 24 hours at 0°C. The International Units are defined by the WHO.

The ophthalmic composition of the invention comprises heparin or a salt thereof, in an amount of from 1000 to 1600 I.U. / mL preferably of from 1100 to 1500 I.U. / mL, more preferably o from 1200 to 1400 I.U / mL, even more preferably from 1250 to 1350 I.U. / mL of the ophthalmic composition. A content of the component (ii) below 1000 I.U. / mL is not preferred since such content may result in insufficient wetting and effect of maintaining corneal cell viability and/or of avoiding an excessive stinging sensation upon application of the ophthalmic composition to the eye.

As mentioned above, "I.U." refers to International Units of heparin activity. The relationship between the mass of the heparin and the international unit depends on the source and preparation methods of the heparin. "USP unit" means the United States Pharmacopeia Unit. The USP unit of heparin is harmonized with the international unit (IU) of heparin, effective October 1, 2009. Therefore, 1 USP unit heparin equals to 1 I.U. heparin. For example, one milligram of heparin obtained from Sigma-Aldrich (H3393) is at least 180 USP units or at least 180 I.U.

The ophthalmic composition of the invention further comprises one or more viscosity regulating agent. Herein, the viscosity regulating agent is other than the heparin or a salt thereof. The viscosity regulating agent of the invention may also be referred to as "thickener", "viscosity improver" or "gelling agent". The viscosity regulating agent is a polymeric hydrophilic compound that is soluble in water, thereby increasing the viscosity of the solution. It has film-building properties when applied to the eye, which protects the cornea and increases the remaining time of the ophthalmic composition on the cornea. The viscosity regulating agent may be a hydroxyalkylated cellulose, alkylated cellulose, chondroitin sulfate, polyacrylic acid, polyvinyl alcohol, polyethylene glycol, polysaccharides, polyvinylpyrrolidone, hyaluronic acid, cross-linked hyaluronic acid, or salts of any of these. The hydroxyalkylated cellulose may be hydroxypropylcellulose or hydroxyethylcellulose. The alkylated cellulose may be methylcellulose or ethylcellulose. Further, a viscosity regulating agent may be hydroxypropylmethylcellulose (hypromellose, HPMC), however, in one embodiment, the ophthalmic composition of the invention does not contain HPMC. Two or more viscosity regulating agents may be combined in the ophthalmic composition of the invention. Among the above viscosity regulating agents, hyaluronic acid or a salt thereof and cross-linked hyaluronic acid or a salt thereof are preferred and hyaluronic acid or a salt thereof is most preferred.

The ophthalmic composition of the invention may comprise the one or more viscosity regulating agent(s), preferably said hyaluronic acid and/or a hyaluronate, in a concentration of from 0.1 to 0.5 % by weight, preferably from 0.15 to 0.45% by weight, more preferably from 0.2 to 0.4 % by weight, and most preferably from 0.25 to 0.35% by weight of the ophthalmic composition. These concentration ranges relate to the sum of viscosity regulating agents, if two or more different are used. Within the above range, the ophthalmic composition such as eye drop formulation is comfortable to apply and suppresses eye irritation. The exact concentration may be chosen so as to reach a desired viscosity of the ophthalmic composition. The viscosity regulating agent may be used alone or in combination of two or more kinds. Among the above, hyaluronic acid and salts thereof is particularly preferred from the view of film stability on the eye and suppression of eye irritation.

The molecular weight of the viscosity regulating agent may be chosen so as to adjust the viscosity of the ophthalmic composition to a desired range. The molecular weight may be within the range of from 100 000 to 10 000 000 Dalton, preferable within the range of from 500 000 to 5 000 000 Dalton, more preferably within the range of from 800 000 to 4 000 000 Dalton, and most preferably within the range of from 1 000 000 to 3 000 000 Dalton.

These molecular weights may be combined with the content of the one or more viscosity regulating agent(s) described above. In this embodiment, the content refers to the amount of the (one or more) viscosity regulating agent in the molecular weight range given. For example, the ophthalmic composition may contain from 0.1 to 0.5 % by weight of one or more viscosity regulating agent, preferably of hyaluronic acid and/or a hyaluronate, having a molecular weight within the range of from 100 000 to 10 000 000 Dalton, preferably within the range of from 500 000 to 5 000 000 Dalton, more preferably within the range of from 800 000 to 4 000 000 Dalton, and most preferably within the range of from 1 000 000 to 3 000 000 Dalton. In another example, the ophthalmic composition may contain from 0.2 to 0.4 % by weight of one or more viscosity regulating agent, preferably of hyaluronic acid and/or a hyaluronate, having a molecular weight within the range of from 100 000 to 10 000 000 Dalton, preferably within the range of from 500 000 to 5 000 000 Dalton, more preferably within the range of from 800 000 to 4 000 000 Dalton, and most preferably within the range of from 1 000 000 to 3 000 000 Dalton. Generally, the molecular weight numbers relate to hyaluronic acid, i.e. do not include the molecular weight of counter cations of hyaluronate, such as sodium.

The weight average molecular weight of the at least one viscosity regulating agent (preferably of the hyaluronic acid or hyaluronate) may be within the range of from 100 000 to 10 000 000 Dalton (100 kDa to 10 MDa), preferable within the range of from 500 000 to 5 000 000 Dalton (500 kDa to 5 MDa), more preferably within the range of from 800 000 to 4 000 000 Dalton (800 kDa to 4 MDa), and most preferably within the range of from 1 000 000 to 3 000 000 Dalton (1 MDa to 3 MDa). The weight average molecular weight may be determined by size exclusion chromatography (SEC) in combination with a multi angle laser light scattering (MALLS) detector (SEC-MALS).

In a preferred embodiment, the ophthalmic composition of the invention comprises hyaluronic acid or a salt thereof as the at least one viscosity regulating agent, and the weight average molecular weight of the hyaluronic acid or salt thereof is within the range of from 100 000 to 10 000 000 Dalton, preferable within the range of from 500 000 to 5 000 000 Dalton, more preferably within the range of from 800 000 to 4 000 000 Dalton, even more preferably within the range of from 1 000 000 to 3 000 000 Dalton, further more preferably within the range of from 1 300 000 to 2 600 000 Dalton, and most preferably within the range of from 1 500 000 to 2 000 000 Dalton. The weight average molecular weight may be determined by SEC-MALS.

As above, these weight average molecular weights may be combined with the content of the one or more viscosity regulating agent(s) described above. For example, the ophthalmic composition may contain from 0.1 to 0.5 % by weight of the one or more viscosity regulating agent (preferably said hyaluronic acid and/or a hyaluronate) and the weight average molecular weight of the at least one viscosity regulating agent is within any one of the ranges given in the previous paragraph. In another example, the ophthalmic composition may contain from 0.2 to 0.4 % by weight of the one or more viscosity regulating agent(s) (preferably said hyaluronic acid and/or a hyaluronate) and the weight average molecular weight of the at least one viscosity regulating agent is within any one of the ranges given in the previous paragraph.

GPC-MALLS is widely used to characterize high molecular weight (MW) polymers, which uses a differential refractometer and a multi-angle laser light scattering detector (MALLS) as detectors for gel permeation chromatogram (GPC). Separation of polymers is achieved by SEC based on different MW. The average molecular weight of an individual polymer is determined by MALLS based the differential scattering extent/angle of molecules of different MW. The GPC-MALLS method allows continuous measurement of the molecular weight and radius of gyration of each fraction separated by GPC. Principles of GPC-MALLS and protocols suited for hyaluronic acid are described by Ueno et al., 1988, Chem. Pharm. Bull. 36, 4971-4975; Wyatt, 1993, Anal. Chim. Acta 272: 1-40; and Wyatt Technologies, 1999, "Light Scattering University DAWN Course Manual" and "DAWN EOS Manual" Wyatt Technology Corporation, Santa Barbara, Calif.

In a preferred embodiment, the ophthalmic composition of the present invention comprises
(i,a) from 4.4 to 6.0 % by weight sodium chloride,
(i,b) from 1.5 to 3.5 % by weight glycerol,
(ii) from 1100 to 1500 I.U. / mL heparin or a salt thereof,
   and
(iii) from 0.1 to 0.5 % by weight hyaluronic acid and/or a hyaluronate.

Amounts given relate to the total weight/volume of the ophthalmic composition.

In an more preferred embodiment, the ophthalmic composition of the present invention comprises
(i,a) from 4.8 to 5.5 % by weight sodium chloride,
(i,b) from 2.0 to 3.0 % by weight glycerol,
(ii) from 1100 to 1500 I.U. / mL heparin or a salt thereof,
   and
(iii) from 0.25 to 0.35 % by weight hyaluronic acid and/or a hyaluronate.
Amounts given relate to the total weight/volume of the ophthalmic composition.

The ophthalmic composition of the present invention may further comprise (iv) an ophthalmically acceptable buffer. Examples of an ophthalmically acceptable buffer include citrate buffer, tris (trometamol) buffer, acetate buffer, borate buffer, and phosphate buffer. Preferred buffers are citrate buffer and tris buffer, most preferred is tris buffer. A content of the buffer in the ophthalmic composition of the invention may be from 5 to 100 mM, preferably from 10 to 60 mM, more preferably from 15 to 50 mM, and even more preferably from 15 to 30 mM.

The ophthalmic composition of the present invention may have a pH value between 6.8 to 7.6, preferably between 7.0 and 7.4, more preferably between 7.1 and 7.3. In a preferred embodiment, the buffer of the ophthalmic composition is tris buffer in a concentration range of from 5 to 50 mM, preferably from 10 to 40 mM, and even more preferably from 10 to 20 mM and the ophthalmic composition has a pH within the range of from 7.1 to 7.3. In an exemplary embodiment, the buffer is 10 to 20 mM tris buffer pH at 7.2.

The dynamic viscosity of the ophthalmic composition of the invention, notably when in the form of eye drops, may be from 20 to 100, preferably from 20 to 70 cP (mPa s) at 20°C determined using a rotational concentric cylinder viscometer at 100 s⁻¹ according to Ph. Eur. 2.2.10. An ophthalmic gel, the dynamic viscosity may be in the range of from 100 to 1000 cP, as measured under these conditions.

The aqueous hyperosmolar ophthalmic composition of the invention may further contain suitable additives or pharmaceutical excipients. Examples of additives or excipients are stabilisers such as EDTA; non-ionic surfactants; or preservatives. Examples of preservatives frequently used in ophthalmic compositions are quaternary ammonium compounds such as benzalkonium chloride, benzethonium chloride, or polyquat. However, preferably, the ophthalmic composition of the invention does not contain a preservative, more preferably it does not contain a quaternary ammonium compound as preservative. Any preservative present may be contained at less than 0.001 % by weight of a preservative, preferably of a quaternary ammonium compound preservative, such as benzalkonium chloride.

The ophthalmic composition of the invention may further contain an active pharmaceutical ingredient, such as an antibiotic, a vitamin, an antiphlogistic agent, an analgesic agent, and/or an agent for decreasing intra-ocular pressure or for treatment of glaucoma. Examples of an antibiotic are chloramphenicol, ofloxacin, norfloxacin, and neomycin. Examples of vitamins are retinol, retinol acetate, retinol palmitate, and tocopherol acetate. An example of an analgesic agent is ibuprofen. Examples of an agent for decreasing intra-ocular pressure or for treatment of glaucoma are bimatoprost, latanoprost, travoprost, brinzolamide, and timolol, or combinations thereof.

The aqueous hyperosmolar ophthalmic composition is for use in ophthalmology. The ophthalmic composition may be for use in a method of treating or reducing corneal swelling or fluid accumulation of any origin, including stromal corneal edema or epithelial corneal edema, or restoring osmotic balance of the cornea, especially after surgical intervention. In one embodiment, the ophthalmic composition is for use in the treatment of corneal edema. The corneal edema may be stromal corneal edema, epithelial corneal edema, corneal osmotic imbalance, post-surgical corneal osmotic imbalance, and corneal swelling or fluid accumulation of any origin.

Furthermore, the present invention provides a method of treating corneal edema, including stromal corneal edema, epithelial corneal edema, corneal osmotic imbalance, especially post-surgical corneal osmotic imbalance, and corneal swelling or fluid accumulation of any origin, the method comprising using the ophthalmic composition according to any one of the preceding claims.

Corneal edema is a common sign of acute or protracted corneal disease. The causes of corneal edema leading to the loss of physical barrier, loss of pump function or increased intraocular pressure, can be classified into four groups: mechanical, toxic, dystrophic and inflammatory. The mechanical cause can be further divided into traumatic and glaucomatous. The traumatic cause could be incidental or surgical. The trauma can be categorized into non-penetrating trauma and penetrating trauma. For example, ocular surgery, which is a penetrating surgical trauma, often leads to corneal edema.

The ophthalmic composition of the present invention is generally applied topically to a subject's eye affected by any of the indications mentioned above. The ophthalmic composition may be applied to an eye, such as the conjunctival sac of an eye, of a subject in need thereof. The composition may be applied in a single dose. However, preferably, it is applied two or more times per day, preferably three to five times per day. It may be applied to an eye one or more times per day over a period of from one day to several weeks (e.g. to four weeks), such as from one day to two weeks or from two days to seven days. For certain indications such as for corneal dystrophy (e.g. Fuchs' dystrophy), the ophthalmic composition may be applied for periods even longer than four weeks, e.g. if treatment by surgery is not possible or desired. In an embodiment, the ophthalmic composition is applied three to five times per day over a period of from 2 to 14 days. If the ophthalmic composition is formulated as an eye drop formulation or ophthalmic gel, it may be applied by dropwise instillation into an eye, such as into the conjunctival sac of an eye, affected by any one of the above indications. In the form of a cream or ointment, the ophthalmic composition may be applied to the surface of an eye, such as into the conjunctival sac of an eye.

The ophthalmic composition of the invention may be formulated, for example, as eye drops, as an ophthalmic gel, ophthalmic cream, or ophthalmic ointment. Preferably, the ophthalmic composition is formulated as eye drops or gel and is applied dropwise to an eye of a subject.

The ophthalmic composition of the invention may be produced through a customary process by dissolving components (i) and (ii) and optionally any desired further component (such as component (iii) and a buffer as component (IV)) in water, adjusting the pH with, for example hydrochloric acid or sodium hydroxide, and adding water up to the desired volume to achieve the aqueous composition with the components in the desired concentrations. Preferably, separate solutions in water of components (ii) and (iii), and optionally excipients such as buffer, may be prepared and added to a solution of component (i) and any further components; then, then adjusting the pH with, for example hydrochloric acid or sodium hydroxide, and adding water up to the desired volume. The resultant solution may then be sterilized, e.g. by sterile filtration, before being packed in suitable plastic containers.

A hyperosmolar ophthalmic gel as the ophthalmic composition of the invention may be produced as follows. Water for injection is heated, sodium chloride as a osmolarity agent is added, optionally a preserving agent and a buffer is added. The solution is stirred and filtered through a microporous membrane. After heating up again, any additional osmotic regulator, if appropriate, and the gel matrix (component (ii) and, if used, component (iii)) are added while hot, the mixture is stirred to reach room temperature. Then water to the desired final volume (e.g. 1000 ml) is added and the mixture is homogenized. A terminal sterilization may be conducted to obtain a uniform and sterile gel.

A hyperosmolar ophthalmic ointment as the ophthalmic composition of the invention may be produced as follows. A hot aqueous phase containing sodium chloride as a osmolarity agent and a thickener (component (ii) and, if used component (iii))is prepared. A hot phase containing fats and an emulsifying agent is prepared separately. The aqueous phase is added to the fat phase and the mixture is homogenized and cooled. A terminal sterilization may be conducted to obtain a uniform and sterile product.

### EXAMPLES

The present invention will next be described in more detail by way of Examples, which should not be construed as limiting the invention thereto.

### Measurement of dynamic viscosity

The determination of the dynamic viscosity for the ophthalmic solutions is carried out by means of a rotational concentric cylinder viscometer (Searle type) at 20°C.

### Materials

- Metallic cylinder supplied with the viscosimeter
- Rotational viscosimeter Visco
- Hook for the UL spindle
- adequate UL spindle

### Method

- Temperature 20°C.
- Pour into the 16ml metallic cylinder the test solution.
- Insert the cylinder in the dedicated section on the base of the viscosimeter.
- Connect the UL spindle to the hook and fix the complex to the stand of the viscosimeter.
- Connect the temperature sensor to the viscosimeter.
- Place the viscosimeter onto the base.
- Turn on the instrument pushing the multi-functional button on the back side.
- In the main menu select the item LEVEL to enter in the instrument level mode to ensure the correct adjustment of the level and if not proper, set the correct level rotating the appropriate stand screws on the base of the instrument.

- Enter in the section MEASURMENT to set the UL spindle.
- Start the measurement from the lower speed (expressed in rpm) set between 100 and 250 rpm. The instrument emits an acoustic signal in case of error, so increase the speed more and more in reference to the displayed value; the analysis result will be the one with the torque value nearest to the 100%.

The measurement is performed three times with the same sample. Reported values are the average of the three measurements.

### Measurement of osmolality

Osmolality is determined by measuring freezing point depression according to Eur. Pharm. 2.2.35. A micro-osmometer Löser Type6/6M was used. The method is carried out as described by the manufacturer as follows:
1. Turn on the instrument and wait for the acoustic signal.
2. Fill the centrifuge tube (1.5 mL) with 100 µl of test solution and place it in the thermistor (temperature dependent resistor).
3. Pull down the thermistor in the cooling hole.
4. The value of the decreasing temperature is displayed. It ensures the progressive freezing. After 1.5 minutes the acoustic signal indicates that the freezing point is reached.
5. Raise completely the freezing needle and insert it into the tube. After 1 second replace the needle to original position.
6. Wait for the acoustic signal and record the value shown on the display.
7. Remove the sample tube.

The measurement for the analytical data determination is performed three times with the same sample/ flask, and we report the average.

The measurement is performed three times with the same sample. Reported values are the average of the three measurements.

### REFERENCE EXAMPLE 1

### Introduction

The EpiOcularTM Eye Irritation Test (EIT) was validated by the European Union Reference Laboratory for Alternatives to Animal Testing (EURL ECVAM) and Cosmetics Europe from 2008 to 2013 (Freeman, S.J., Alépée N., Barroso, J., Cole, T., Compagnoni, A., Rubingh, C., Eskes, C., Lammers, J., McNamee, P., Pfannenbecker, U., Zuang, V. (2010). Prospective Validation Study of Reconstructed Human Tissue Models for Eye Irritation Testing. ALTEX 27, Special Issue 2010, 261-266; EC EURL ECVAM (2014). Validation Study Report on the EURL ECVAM - Cosmetics Europe Prospective Validation Study of Reconstructed Human Corneal Epithelium-Based Test Methods for Identifying Chemicals not Requiring Classification and Labelling for Serious Eye Damage/Eye Irritation Testing; EC EURL ECVAM (2014) Eye Irritation In Vitro Assay Validation: Selection of Test Item Chemicals (EpiOcular™ Eye Irritation Test and SkinEthic™ Human Cornea Epithelium). Validation Management Group report; TNO (2015). Eye Irritation Validation Study on Human Tissue Models: Statistical Analysis and Reporting on the EpiOcular™ EIT. TNO Report TNO2013 R10396 Final, pp. 165; EC EURL ECVAM (2014) Eye Irritation Validation Study (EIVS): Statistical Analysis of the Data Generated Under SOP ver 8.0 of EpiOcular™ EIT (Solid Test Substances, Laboratory Beiersdorf), pp. 21).

From this validation study and its independent peer review (EC EURL ECVAM (2014). Recommendation on the Use of the EpiOcular^{™} Eye Irritation Test (EIT) for Identifying Chemicals not Requiring Classification and Labelling for Serious Eye Damage/Eye Irritation According to UN GHS) it was concluded that the EpiOcular^{™} EIT is able to correctly identify chemicals (both substances and mixtures) not requiring classification and labelling for eye irritation or serious eye damage according to UN GHS [7] (i.e. "No Category"), and the test method was recommended as scientifically valid for this purpose. This in vitro method is designed to evaluate the eye hazard potential of a test chemical not requiring classification for eye irritation or serious eye damage in accordance with UN GHS [7] based on its ability to induce cytotoxicity in a reconstructed human cornea-like epithelium (RhCE) tissue construct after topical application, expressed as the reduction of mitochondrial dehydrogenase activity measured by formazan production from 3-(4,5-dimethylthiazol-2-yl)-2,5-diphenyltetrazolium bromide (MTT) (Mosmann, T. (1983). Rapid colorimetric assay for cellular growth and survival: application to proliferation and cytotoxicity assays. J. Immunol. Meth. 65: 5563). This test uses the three-dimensional RhCE EpiOcular^{™} (MatTek). It consists of normal, human-derived epidermal keratinocytes and mimics the histological, morphological, biochemical and physiological properties of the human corneal epithelium. The MatTek EpiOcular^{™} model has been widely used as a research and testing model for many years (Kaluzhny, Y. et al. (2011) Development of the EpiOcular™ Eye Irritation Test for Hazard Identification and Labelling of Eye Irritating Chemicals in Response to the Requirements of the EU Cosmetics Directive and REACH Legislation. ATLA 39, 339-364). Ocular irritation potential is predicted by the relative viability of the tissue after a single exposure to the test substance. Relative viability is determined by measuring the MTT dye to formazan conversion by the EpiOcular^{™} tissue construct after topical exposure to the test substance.

### Materials and Methods

### Characterisation of the Test Item

Name: ODM5 (Horus Pharma)
Batch No.: ODM0818
Aggregate State at RT: liquid
Storage Conditions: room temperature
Type of Material: liquid eye drops

ODM5 as the test item has the following composition:
5% NaCl,
0.15% sodium hyaluronate,
0.25% hydroxypropylmethylcellulose,
trisodium citrate dihydrate
citric acid monohydrate, and
water
osmolarity: 1750 mOsm/L.

### Preparation and Application of the Test Item

The test item was applied undiluted. 50 µL (83.3 µL/cm²) of the test item were dispensed directly atop the EpiOcular^{™} tissue. The test item was spread to match the size of the tissue.

### Controls

Controls were set up in parallel to the test item cultures in order to confirm the validity of the test. Negative Control: Distilled water (aqua dest.; Sigma, Lot No.: RNBG4913). Positive Control: Methyl acetate (CAS No. 79-20-9; Merck, Lot No.: S6943111)

### Dose Groups

1. Negative Control
2. Positive Control
3. Test Item

The test was performed on a total of 2 tissues per dose group.

### Test System

The test was carried out with the EpiOcular^{™} reconstructed human cornea-line epithelium (RhCE) model (MatTek). The model consists of normal, human-derived epidermal keratinocytes which have been cultured to form a stratified, highly differentiated squamous epithelium morphologically similar to that found in a human cornea. The EpiOcular^{™} RhCE tissue construct consists of at least 3 viable layers of cells and a non-keratinized surface, showing a cornea-like structure analogous to that found *in vivo.*

### Provided Materials

The EpiOcular^{™} tissues were provided as kits (e.g. OCL-200-EIT; MatTek), consisting of the following components relevant for this study:
1x sealed 24-well plate containing 24 inserts with EpiOcular^{™} tissues on agarose (Lot No.: 30624)
1x bottle EpiOcular^{™} assay medium (Lot No.: 090219ISA) 1x bottle Ca²⁺/Mg²⁺-free DPBS buffer (Lot No.: 052819ALA)

### Further reagents

### MTT solution

- MTT stock solution: 5 mg/mL MTT (VWR; Lot No.: 1811156332) in PBS (Gibco; Lot No.: 2098592 [main exp.], 2026787 [pre-test])
- MTT medium: MTT stock solution was diluted 1 + 4 with DMEM-based medium (final concentration 1.0 mg/mL)
   **Isopropanol** (Applichem; Lot No.: 0001245289 ),
   **Aqua dest.** (Sigma Aldrich, Lot. No.: RNBG3520 [pre-test])

### Experimental Procedure

Upon receipt of the EpiOcular^{™}, the tissues were equilibrated in the 24-well shipment plate to room temperature for about 15 min. Then, the EpiOcular^{™} tissues were transferred into 6-well plates containing 1 mL pre-warmed assay medium per well and incubated for 1 h in a humidified incubator at 37 ± 2°C, 5.0% CO₂ / 95% air. Then, the inserts were transferred into new 6-well plates containing 1 mL fresh assay medium per well and pre-incubated in a humidified incubator at 37 ± 2°C, 5.0% CO₂ /95%air for 16-24 h.

After the overnight incubation the tissues were pre-treated with 20 µL of (Dulbecco's phosphate-buffered saline) DPBS-buffer and incubated for 30 ± 2 min in a humidified incubator at 37 ± 2°C, 5.0% CO₂ / 95% air to mimic the wet conditions of the human eye.

Afterwards, the tissues were treated with each dose group in duplicate, starting with the negative and positive control. Then the 6-well plate(s) were incubated for 30 ± 2 min at 37 ± 2 °C, 5.0% CO₂ / 95% air. At the end of the exposure period, the test item and control substances were removed by extensively rinsing the tissue with DPBS (Dulbecco's phosphate buffered saline). Excess DPBS was removed by decanting the insert and blotting bottom with blotting paper. After rinsing, the inserts were transferred to and immersed in a prepared 12-well "post-soak plate", containing 5 mL fresh pre-warmed assay medium per well and incubated for 12 ± 2 min at room temperature. Afterwards, the inserts were removed from the assay medium, the medium was decanted off the tissue and the tissues were blotted on blotting paper. The inserts were transferred to a new 6-well plate (post-treatment plate) containing 1 mL pre-warmed assay medium. The tissues were incubated for 120 ± 15 min at 37 ± 2°C, 5.0% CO₂ / 95% air.

After this incubation period excess medium was removed by blotting bottom on absorbent paper before the inserts were transferred in a prepared 24-well "MTT assay plate" containing 0.3 mL pre- warmed MTT medium and further incubated for 3 h ± 15 min at 37 ± 2°C, 5.0% CO₂/ 95% air.

After the 3 h MTT incubation period the inserts were removed, the bottom of the inserts blotted on blotting paper, and then transferred into new 24-well "extraction plates", containing 2 mL of isopropanol. The extraction plates were sealed to inhibit isopropanol evaporation. Extraction was carried out immediately by shaking on an orbital plate shaker for 2 - 3 h at room temperature. At the end of the extraction period the tissues were pierced and the liquid within each insert was decanted into the well from which it was taken.

Then the inserts were discarded and the extracts were mixed three times using a pipette. If any visible cell/tissue fragments were in suspension, extracts were centrifuged to eliminate the fragments and avoid further possible interference with the absorbance readings.

For each tissue 2 × 200 µL aliquots of the extract were transferred into a 96-well plate and OD was measured at 570 nm using a filter band pass of maximum ± 30 nm in a plate spectrophotometer using isopropanol as a blank.

### Test Acceptance Criteria

The test meets acceptance criteria if:
- mean absolute OD₅₇₀ₙₘ of the negative control is > 0.8 and < 2.5
- mean relative tissue viability of the positive control is < 50%
- relative tissue viability difference of replicate tissues is < 20%.

### Results

**Table 1: Results of Test Item ODM5**

| **Name** | **Negative Control** | | **Positive Control** | | **Test Item** | |
|---|---|---|---|---|---|---|
| **Replicate Tissue** | **1** | **2** | **1** | **2** | **1** | **2** |
| **Absolute OD₅₇₀** | 1.722 | 1.725 | 0.651 | 0.667 | 1.723 | 1.621 |
| | 1.756 | 1.759 | 0.657 | 0.677 | 1.774 | 1.671 |
| **Mean Absolute OD₅₇₀** | 1.740**** | | 0.663 | | 1.697 | |
| **OD₅₇₀ (Blank Corrected)** | 1.675 | 1.678 | 0.604 | 0.621 | 1.676 | 1.575 |
| | 1.710 | 1.712 | 0.610 | 0.630 | 1.727 | 1.625 |
| **Mean OD₅₇₀ of the Duplicates (Blank Corrected)** | 1.693 | 1.695 | 0.607 | 0.626 | 1.702 | 1.600 |
| **Total Mean OD₅₇₀ of the 2 Replicate Tissues (Blank Corrected)** | 1.694* | | 0.616 | | 1.651 | |
| **TOD_{Π}** | - | | - | | 1.644 | |
| **SD of Mean OD₅₇₀ of the Duplicates (Blank Corrected)** | 0.002 | | 0.013 | | 0.072 | |
| **Relative Tissue Viability [%]** | 99.9 | 100.1 | 35.9 | 36.9 | 100.5 | 94.4 |
| **Relative Tissue Viability Difference [%]***** | 0.2 | | 1.1 | | 6.0 | |
| **Mean Relative Tissue Viability [%]** | **100.0** | | **36.4**** | | **97.5** | |
| **Mean Tissue Viability [%] - NSC_{living} corrected** | - | | - | | **97.1** | |

| | | | | | | |
|---|---|---|---|---|---|---|
| * Corrected mean OD₅₇₀ of the negative control corresponds to 100% absolute tissue viability ** Mean relative tissue viability of the positive control is < 50% *** Relative tissue viability difference of replicate tissues is < 20% **** Mean absolute OD₅₇₀ of the negative control is ≥ 0.8 and ≤ 2.5 | | | | | | |

### EXAMPLE 1

A test was carried out as described in Reference Example 1, except that the following eye drop composition was used as the Test Item instead of ODM5:

### Test Item (of the present invention) has the following composition:

Composition per mL of eye drop solution:

| **Component** | **Quality** | **Quantity [mg]** |
|---|---|---|
| Glycerol 85% | Ph. Eur. | 25.0 |
| Sodium chloride | Ph. Eur. | 50.0 |
| Sodium hyaluronate | Ph. Eur. | 3.0 |
| Heparin sodium | Ph. Eur. | 6.5 (≙1300 I. U.) |
| Trometamol (Tris) | Ph. Eur. | 5.0 |
| Sodium acetate, trihydrate | Ph. Eur. | 16.5 |
| Purified water | Ph. Eur. | 942 |
| Acetic acid | Ph. Eur. | q. s. |

The molecular weight of the sodium hyaluronate was 1.5 to 1.75 MDa according to the certificate of analysis, and was determined to be 1.69 MDa by SEC-MALS.

The Test Item was manufactured as follow: Two thirds of the purified water and glycerol are charged in a tank and stirred for 10 minutes. Trometamol, sodium acetate, sodium hyaluronate, heparin sodium and sodium chloride are weighed separately and pre-mixed in a disposable bag. The solid mixture is added to the solution and stirred for 3 hours. The pH is adjusted with acetic acid to 7.3 and water is filled up to reach the final volume. The solution is sterile-filtered through a 0.20 µm filter and filled into a suitable eye drop flask (e. g. made of HDPE).

Analysis:

| | |
|---|---|
| pH: | 7.30 |
| measured Osmolality (mOsmol/kg): | 2242 |
| Viscosity at 20°C (cP): | 43.30 |
| Relative density (g/cm³): 1.05 | |

**Table 2: Results of Test Item of the invention**

| **Name** | **Negative Control** | | **Positive Control** | | **Test Item** | |
|---|---|---|---|---|---|---|
| **Replicate Tissue** | **1** | **2** | **1** | **2** | **1** | **2** |
| **Absolute OD₅₇₀** | 1.722 | 1.725 | 0.651 | 0.667 | 1.780 | 1.826 |
| | 1.756 | 1.759 | 0.657 | 0.677 | 1.841 | 1.887 |
| **Mean Absolute OD₅₇₀** | 1.740**** | | 0.663 | | 1.834 | |
| **OD₅₇₀ (Blank Corrected)** | 1.675 | 1.678 | 0.604 | 0.621 | 1.734 | 1.780 |
| | 1.710 | 1.712 | 0.610 | 0.630 | 1.794 | 1.841 |
| **Mean OD₅₇₀ of the Duplicates (Blank Corrected)** | 1.693 | 1.695 | 0.607 | 0.626 | 1.764 | 1.810 |
| **Total Mean OD₅₇₀ of the 2 Replicate Tissues (Blank Corrected)** | 1.694* | | 0.616 | | 1.787 | |
| **SD of Mean OD₅₇₀ of the Duplicates (Blank Corrected)** | 0.002 | | 0.013 | | 0.033 | |
| **Relative Tissue Viability [%]** | 99.9 | 100.1 | 35.9 | 36.9 | 104.2 | 106.9 |
| **Relative Tissue Viability Difference [%]***** | 0.2 | | 1.1 | | 2.7 | |
| **Mean Relative Tissue Viability [%]** | **100.0** | | **36.4**** | | **105.5** | |

| | | | | | | |
|---|---|---|---|---|---|---|
| * Corrected mean OD₅₇₀ of the negative control corresponds to 100% absolute tissue viability ** Mean relative tissue viability of the positive control is < 50% *** Relative tissue viability difference of replicate tissues is < 20% **** Mean absolute OD₅₇₀ of the negative control is ≥ 0.8 and ≤ 2.5 | | | | | | |

A comparison of the of cell viability between Reference Example 1 and Example 1 shows that the ophthalmic composition of the invention has an improved mean relative tissue viability compared to the that of the commercial product ODM5 of Reference Example 1, although the ophthalmic composition of Example 1 has a higher osmolality than ODM5. From the higher osmolality, one would have expected a lower cell viability or higher irritation.

## Claims

1. A hyperosmolar ophthalmic composition comprising:
(i) at least one osmolarity agent,
(ii) heparin or a salt thereof in an amount of from 1000 to 1600 I.U. / mL of the ophthalmic composition, and
(iii) at least one viscosity regulating agent,
wherein
(A) the total osmolarity of the ophthalmic composition is at least 1500 mOsm/L, preferably at least 1800 mOsm/L; or
(B) the ophthalmic composition contains, as osmolarity agent (i), at least 4.4 % by weight sodium chloride.

2. The ophthalmic composition according to claim 1, wherein the (i) at least one osmolarity agent of (A) is selected from the group consisting of sodium chloride, potassium chloride, trehalose, and sugar alcohols.

3. The ophthalmic composition according to claim 2, wherein the sugar alcohol is selected from the group consisting of glycerol, mannitol, and sorbitol.

4. The ophthalmic composition according to any one of claims 1 to 3, wherein the (i) at least one osmolarity agent is or comprises sodium chloride and one or more sugar alcohol.

5. The ophthalmic composition according to any one of claims 2 to 4, wherein the sugar alcohol is glycerol, preferably wherein the ophthalmic composition comprises sodium chloride and glycerol as osmolarity agents (i).

6. The ophthalmic composition according to any one of claims 1 to 5, comprising, as osmolarity agents (i), sodium chloride in a concentration of from 4.4 to 6.5 % by weight, preferably from 4.6 to 6.0 % by weight, more preferably from 4.8 to 5.5 % by weight, and even more preferably about 5 % by weight.

7. The ophthalmic composition according to any one of claims 1 to 6, comprising, as osmolarity agent (i), glycerol in an amount of from 1.5 to 3.5 %, preferably from 2.0 to 3.0 %, and even more preferably from 2.3 to 2.7 % by weight.

8. The ophthalmic composition according to any one of claims 1 to 7, containing (ii) heparin or a salt thereof in an amount of from 1100 to 1500 I.U. / mL, preferably of from 1200 to 1400 I.U / mL, more preferably of from 1250 to 1350 I.U. / mL of the ophthalmic composition.

9. The ophthalmic composition according to any one of claims 1 to 8, wherein the (iii) one or more viscosity regulating agent is selected from the group consisting of a hydroxyalkylated cellulose, chondroitin sulfate, polyacrylic acid, polyvinyl alcohol, polyethylene glycol, polysaccharides, polyvinylpyrrolidone, hyaluronic acid, cross-linked hyaluronic acid, and salts thereof, preferably wherein the (iii) one or more viscosity regulating agent is or comprises hyaluronic acid and/or a hyaluronate.

10. The ophthalmic composition according to any one of claims 1 to 9, comprising said (iii) one or more viscosity regulating agent, preferably hyaluronic acid and/or hyaluronate, in a concentration of from 0.1 to 0.5 %, preferably from 0.15 to 0.45%, more preferably from 0.2 to 0.4 %, and most preferably from 0.25 to 0.35% by weight.

11. The ophthalmic composition according to any one of claims 1 to 10, further comprising (iv) a buffer, preferably wherein the (iv) buffer is selected from the group consisting of a tris buffer and citrate buffer, preferably the buffer is tris buffer.

12. The ophthalmic composition according to any one of claims 1 to 11, having a pH of from 6.8 to 7.6; and/or a dynamic viscosity of from 20 to 70 cP (mPa s) at 20°C using a rotational viscosimeter.

13. An ophthalmic composition according any one of the preceding claims for use in ophthalmology, preferably for use in a method of treating or reducing corneal swelling or fluid accumulation of any origin, including cataract surgery, corneal transplantation, stromal corneal edema or epithelial corneal edema, or for restoring osmotic balance of the cornea, especially after surgical intervention such as cataract surgery or corneal transplantation.

## Patentansprüche

1. Hyperosmolare ophthalmische Zusammensetzung, umfassend
(i) wenigstens ein Osmolaritätsmittel,
(ii) Heparin oder ein Salz desselben in einer Menge von 1.000 bis 1.600 IE/ml der ophthalmischen Zusammensetzung und
(iii) wenigstens ein Mittel zur Regulierung der Viskosität,
wobei (A) die Gesamtosmolarität der ophthalmischen Zusammensetzung mindestens 1.500 mOsm/l, bevorzugt mindestens 1.800 mOsm/l beträgt; oder
(B) die ophthalmische Zusammensetzung als das Osmolaritätsmittel (i) mindestens 4,4 Gewichtsprozent Natriumchlorid enthält.

2. Die ophthalmische Zusammensetzung nach Anspruch 1, wobei das wenigstens eine Osmolaritätsmittel (i) von (A) ausgewählt ist aus der Gruppe, bestehend aus Natriumchlorid, Kaliumchlorid, Trehalose und Zuckeralkoholen.

3. Die ophthalmische Zusammensetzung nach Anspruch 2, wobei der Zuckeralkohol ausgewählt ist aus der Gruppe, bestehend aus Glycerin, Mannit und Sorbit.

4. Die ophthalmische Zusammensetzung nach einem der Ansprüche 1 bis 3, wobei das wenigstens eine Osmolaritätsmittel (i) Natriumchlorid und ein oder mehrere Zuckeralkohole ist oder umfasst.

5. Die ophthalmische Zusammensetzung nach einem der Ansprüche 2 bis 4, wobei der Zuckeralkohol Glycerol ist, wobei die ophthalmische Zusammensetzung bevorzugt Natriumchlorid und Glycerol als Osmolaritätsmittel (i) umfasst.

6. Die ophthalmische Zusammensetzung nach einem der Ansprüche 1 bis 5, umfassend als Osmolaritätsmittel (i) Natriumchlorid in einer Konzentration von 4,4 bis 6,5 Gewichtsprozent, bevorzugt von 4,6 bis 6,0 Gewichtsprozent, bevorzugter von 4,8 bis 5,5 Gewichtsprozent und am stärksten bevorzugt etwa 5 Gewichtsprozent.

7. Die ophthalmische Zusammensetzung nach einem der Ansprüche 1 bis 6, umfassend als Osmolaritätsmittel (i) Glycerol in einer Menge von 1,5 bis 3,5 Gewichtsprozent, bevorzugt von 2,0 bis 3,0 Gewichtsprozent und bevorzugter von 2,3 bis 2,7

8. Die ophthalmische Zusammensetzung nach einem der Ansprüche 1 bis 7, enthaltend (ii) Heparin oder ein Salz desselben in einer Menge von 1.100 bis 1.500 IE/ml, bevorzugt von 1.200 bis 1.400 IE/ml, bevorzugter von 1.250 bis 1.350 IE/ml der ophtalmischen Zusammensetzung.

9. Die ophthalmische Zusammensetzung nach einem der Ansprüche 1 bis 8, wobei das eine oder die mehreren Mittel zur Regulierung der Viskosität (iii) ausgewählt sind aus der Gruppe bestehend aus hydroxyalkylierter Cellulose, Chondroitinsulfat, Polyacrylsäure, Polyvinylalkohol, Polyethylenglycol, Polysacchariden, Polyvinylpyrrolidon, Hyaluronsäure, vernetzter Hyaluronsäure und Salzen derselben, wobei das eine oder die mehreren Mittel zur Regulierung der Viskosität (iii) bevorzugt Hyaluronsäure und/oder ein Hyaluronat sind oder diese umfassen.

10. Die ophthalmische Zusammensetzung nach einem der Ansprüche 1 bis 9, umfassend ein oder mehrere Mittel zur Regulierung der Viskosität (iii), bevorzugt Hyaluronsäure und/oder Hyaluronat in einer Konzentration von 0,1 bis 0,5 Gewichtsprozent, bevorzugt von 0,15 bis 0,45 Gewichtsprozent, stärker bevorzugt von 0,2 bis 0,4 Gewichtsprozent und am stärksten bevorzugt von 0,25 bis 0,35 Gewichtsprozent.

11. Die ophthalmische Zusammensetzung nach einem der Ansprüche 1 bis 10, ferner (iv) einen Puffer umfassend, wobei der Puffer (iv) ausgewählt ist aus der Gruppe, bestehend aus einem Tris-Puffer und Citratpuffer, bevorzugt ist der Puffer Tris-Puffer.

12. Die ophthalmische Zusammensetzung nach einem der Ansprüche 1 bis 11, die einen pH-Wert von 6,8 bis 7,6 aufweist und/oder eine dynamische Viskosität von 20 bis 70 cP (mPa s) bei 20°C unter Verwendung eines Rotationsviskosimeters.

13. Die ophthalmische Zusammensetzung nach einem der vorhergehenden Ansprüche zur Verwendung in der Ophthalmologie, bevorzugt zur Verwendung in einem Verfahren zur Behandlung oder Reduzierung von Hornhautschwellungen oder Flüssigkeitsansammlungen jeglichen Ursprungs, einschließlich Kataraktchirurgie, Hornhauttransplantation, stromalem Hornhautödem oder epithelialem Hornhautödem, oder zur Wiederherstellung des osmotischen Gleichgewichts der Hornhaut, insbesondere nach chirurgischen Eingriffen, wie einer Kataraktoperation oder einer Hornhauttransplantation.

## Revendications

1. Composition hyperosmolaire ophtalmique comprenant :
(i) au moins un agent d'osmolarité,
(ii) de l'héparine ou un sel de celle-ci en une quantité de 1000 à 1600 U.I./ml de la composition ophtalmique, et
(iii) au moins un agent régulateur de viscosité,
dans laquelle :
(A) l'osmolarité totale de la composition ophtalmique est d'au moins 1500 mOsm/l, de préférence d'au moins 1800 mOsm/l ; ou
(B) la composition ophtalmique contient, en tant qu'agent d'osmolarité (i), au moins 4,4 % en poids de chlorure de sodium.

2. Composition ophtalmique selon la revendication 1, dans laquelle (i) l'au moins un agent d'osmolarité de (A) est choisi dans le groupe constitué par le chlorure de sodium, le chlorure de potassium, le tréhalose, et les alcools de sucre.

3. Composition ophtalmiques selon la revendication 2, dans laquelle l'alcool de sucre est choisi dans le groupe constitué par le glycérol, le mannitol, et le sorbitol.

4. Composition ophtalmique selon l'une quelconque des revendications 1 à 3, dans laquelle (i) l'au moins un agent d'osmolarité de (A) est ou comprend du chlorure de sodium et un ou plusieurs alcools de sucre.

5. composition ophtalmique selon l'une quelconque des revendications 2 à 4, dans laquelle l'alcool de sucre est le glycérol, de préférence dans laquelle la composition ophtalmique comprend du chlorure de sodium et du glycérol en tant qu'agents d'osmolarité (i).

6. Composition ophtalmique selon l'une quelconque des revendications 1 à 5, comprenant, en tant qu'agents d'osmolarité (i), du chlorure de sodium a une concentration de 4,4 à 6,5 % en poids, de préférence de 4,6 à 6,0 % en poids, plus préférablement de 4,8 à 5,5 % en poids, et encore plus préférablement d'environ 5 % en poids.

7. Composition ophtalmique selon l'une quelconque des revendications 1 à 6, comprenant, en tant qu'agent d'osmolarité (i), du glycérol en une quantité de 1,5 à 3,5 %, de préférence de 2,0 à 3,0 %, et encore plus préférablement de 2,3 à 2,7 % en poids.

8. Composition ophtalmique selon l'une quelconque des revendications 1 à 7, contenant (ii) de l'héparine ou un sel de celle-ci en une quantité de 1100 à 1500 U.I./ml, de préférence de 1200 à 1400 U.I./ml, plus préférablement de 1250 à 1350 U.I./ml de la composition ophtalmique.

9. Composition ophtalmique selon l'une quelconque des revendications 1 à 8, dans laquelle (iii) le ou les agents régulateur de viscosité sont choisis dans le groupe constitué par une cellulose hydroxyalkylée, le sulfate de chondroïtine, un poly(acide acrylique), un poly(alcool vinylique), un polyéthylèneglycol, les polysaccharides, une polyvinylpyrrolidone, l'acide hyaluronique, un acide hyaluronique réticulé, et leurs sels, de préférence dans laquelle (iii) le ou les agents régulateur de viscosité sont ou comprennent de l'acide hyaluronique et/ou un hyaluronate.

10. Composition ophtalmique selon l'une quelconque des revendications 1 à 9, comprenant lesdits (iii) un ou plusieurs agents régulateurs de viscosité, de préférence de l'acide hyaluronique et/ou un hyaluronate, à une concentration de 0,1 à 0,5 %, de préférence de 0,15 à 0,45 %, plus préférablement de 0,2 à 0,4 %, et le plus préférablement de 0,25 à 0,35 % en poids.

11. Composition ophtalmique selon l'une quelconque des revendications 1 à 10, comprenant en outre (iv) un tampon, de préférence dans laquelle le tampon (iv) est choisi dans le groupe constitué par un tampon Tris et un tampon citrate, de préférence le tampon est un tampon Tris.

12. Composition ophtalmique selon l'une quelconque des revendications 1 à 11, ayant un pH de 6,8 à 7,6 ; et/ou une viscosité dynamique de 20 à 70 cP (mPa.s) à 20°C par utilisation d'un viscosimètre rotationnel.

13. Composition ophtalmique selon l'une quelconque des revendications précédentes, pour une utilisation en ophtalmologie, de préférence pour une utilisation dans une méthode de traitement ou de réduction d'un gonflement de la cornée ou d'une accumulation de fluides de n'importe quelle origine, y compris une opération de la cataracte, une greffe de cornée, un oedème stromal de la cornée ou un œdème épithélial de la cornée, ou pour rétablir l'équilibre osmotique de la cornée, en particulier après une intervention chirurgicale telle qu'une opération de la cataracte ou une greffe de cornée.
